Europäisches Patentamt

European Patent Office

Office européen des brevets

(19)

(11) Numéro de publication : **0 188 441**
**B1**

# (12) FASCICULE DE BREVET EUROPÉEN

(45) Date de publication du fascicule du brevet :
**07.09.88**

(21) Numéro de dépôt : **85902578.5**

(22) Date de dépôt : **13.06.85**

(86) Numéro de dépôt international :
**PCT/FR 85/00148**

(87) Numéro de publication internationale :
**WO/8600220 (16.01.86 Gazette 86/02)**

(51) Int. Cl.⁴ : **A 61 F 13/02**

## (54) PANSEMENT ADHESIF PERMETTANT UNE APPLICATION AISEE SUR LA PEAU.

(30) Priorité : **25.06.84 FR 8409963**

(43) Date de publication de la demande :
**30.07.86 Bulletin 86/31**

(45) Mention de la délivrance du brevet :
**07.09.88 Bulletin 88/36**

(84) Etats contractants désignés :
**AT BE CH DE FR GB IT LI LU NL SE**

(56) Documents cités :
**FR-A- 1 314 185**
**US-A- 2 473 062**
**US-A- 4 304 333**
**US-A- 4 402 696**

(73) Titulaire : **KAYSERSBERG SA**
**Route de Lapoutroie**
**F-68240 Kaysersberg (FR)**

(72) Inventeur : **MAZARS, Paul**
**Amfreville sur Iton**
**F-27400 Louviers (FR)**
Inventeur : **BARRETEAU, Jean-Pierre**
**F-27110 St Aubin d'Ecrosville**
**F-27110 St Aubin d'Ecrosville (FR)**

(74) Mandataire : **David, Daniel**
**KAYSERSBERG 54, avenue Hoche**
**F-75008 Paris (FR)**

**Description**

La présente invention concerne le domaine des articles adhésifs utiles pour recouvrir la peau d'humains et d'animaux.

Elle a pour objet un pansement adhésif qui au moyen d'une combinaison particulière d'éléments permet une application aisée sur la peau.

Selon le préambule de l'invention le pansement est constitué d'un film plastique recouvert sur une face d'un adhésif sensible à la pression et d'un support anti-adhérent protégeant cet adhésif avant utilisation.

Etat de la technique

Ces articles adhésifs et leur application comme pansements sont connus depuis longtemps et ont fait l'objet de nombreux dépôts de brevets.

Ainsi les brevets français 1025117, 1288390 et 2012584 décrivent des pansements adhésifs constitués d'un film plastique, synthétique ou naturel, pourvu sur une surface d'un adhésif sensible à la pression et d'un support antiadhérent, comme du papier siliconé, protégeant l'adhésif. Le film plastique, très mince (de l'ordre de 20 microns) et sans aucune rigidité, a tendance à adhérer sur lui-même en formant des plis lorsque le support est retiré avant le positionnement sur la peau. Ces plis rendent difficile, voire impossible l'utilisation du pansement, qui doit épouser la forme de la peau pour assurer un bon isolement de la plaie vis-à-vis du milieu extérieur.

Ainsi, plusieurs solutions ont été proposées afin de remédier à cet inconvénient.

L'une d'entre elles est décrite dans la demande de brevet européen N° 0051935. Elle consiste à adjoindre à la face du film non recouverte par l'adhésif un support rigide ayant une faible adhésion pour le film plastique par rapport à l'adhésif qui permet de maintenir le film sur la peau. Ainsi lorsque, avant positionnement, le support protégeant l'adhésif est retiré la planéité du film plastique est conservée grâce au support rigide. Ensuite, lorsque le film est appliqué sur la peau il est possible d'enlever le support rigide du fait de sa faible adhérence au film plastique. Ce dispositif nécessite toutefois l'emploi d'un support supplémentaire.

Il en est de même pour la demande de brevet européen N° 0081890.

Une autre solution décrite dans la demande de brevet européen N° 0081889 propose un pansement adhésif dont le support protecteur ainsi que le film sont pourvus de fenêtres centrales séparées de leur zone extérieure respective ainsi délimitée par une ligne de rupture. Lors du positionnement, on enlève d'abord la fenêtre centrale du support protecteur, on applique le pansement sur la peau et on sépare ensuite les zones externes, du support et du film, de la fenêtre centrale du film qui reste appliquée à la peau.

Ce dispositif est fort complexe car il implique la réalisation de lignes de rupture à la fois dans le support protecteur et dans le film. En outre, la zone externe du film plastique est inutilisée puisqu'elle est retirée avec le reste du support.

L'objet de l'invention est de proposer un dispositif simple tant au niveau de sa réalisation que de son utilisation. Il permet notamment son application par une seule personne. Ce dispositif est, en outre, économique puisqu'il n'exige pas l'élimination d'une partie du film plastique.

Description générale de l'invention

Le pansement adhésif est caractérisé en ce que :
— il est replié sur lui-même en deux parties, du côté du support protecteur, selon une ligne de pliage transversale,
— la partie support protecteur correspondant à une des deux parties du pansement est munie d'une ligne de rupture sensiblement parallèle à la ligne de pliage,
— la zone du support comprise entre la ligne de rupture et la ligne de pliage est pourvue sur sa face externe d'un adhésif qui agit de manière à ce que ladite zone adhère plus fortement à la zone correspondante de l'autre partie du support qu'au film plastique.

Le pansement peut être replié sur lui-même en deux parties égales. Mais il pourra être avantageux que l'une des deux parties déborde légèrement sur l'autre de manière à faciliter la préhension.

La ligne de rupture peut être une ligne de faiblesse obtenue par perforation ou, de manière préférée, une ligne de découpe ce qui permet une séparation sans risque de déchirement.

La zone du pansement qui correspond à la zone du support comprise entre la ligne de rupture et la ligne de pliage a pour fonction, une fois qu'elle a été découverte, de permettre le positionnement du pansement. Ainsi l'importance de la zone en question du support pourra varier de manière assez large sans pour cela sortir de l'invention.

En général, la surface occupée par cette zone sera relativement minime dans le cas de petits pansements et plus importante pour les grands pansements.

L'adhésif qui lie les deux zones de support peut être choisi de manière non limitative parmi les adhésifs double face, les adhésifs thermodurcissables ou les adhésifs permanents encore appelés dans la langue anglaise adhésif sensible à la pression. Ce qui est important, avant tout, c'est que la liaison entre les deux zones du support soit plus forte que la liaison support-film. L'homme de métier pourra aisément choisir au vu de ces indications l'adhésif qui convient. Il faut d'ailleurs remarquer, à ce propos, que cela peut dépendre de la nature de la face externe du support. Ainsi généralement le support est constitué d'un papier revêtu sur sa face interne de silicone. Dans ce cas, le silicone étant un antiadhérent il sera possible de lier les deux zones du support par le même adhésif qui recouvre le film puisque la liaison film-papier siliconé sera toujours moins forte que la liaison papier-papier. On préférera toutefois les adhésifs thermodurcissables. Il est possible encore d'ajouter au dispositif qui vient d'être décrit des languettes de préhension assurant un meilleur décollement, d'une part de l'élément du support constitué par une des parties et la zone comprise entre les lignes de pliage et de rupture, d'autre part du complément de l'autre partie du support.

Description d'un mode de réalisation préféré

— la figure 1 est une vue en perspective d'un pansement selon l'invention
— la figure 2 est une vue en perspective du même pansement déplié
— la figure 3 est une vue en perspective du pansement partiellement appliqué sur la peau.

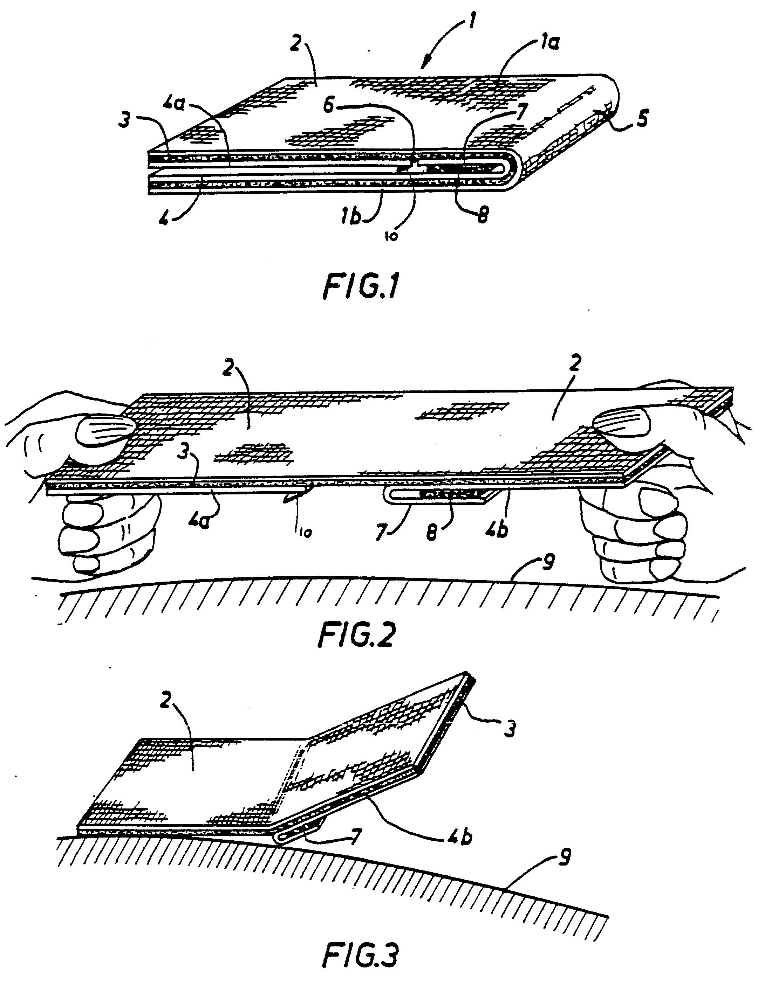

Selon la figure 1, le pansement 1 de forme rectangulaire est constitué d'un film plastique 2 perméable à la vapeur d'eau comme un film de polyuréthane thermoplastique ou de polyamide ou de tout autre matériau ayant les mêmes propriétés élastiques. Ce film est recouvert sur une surface d'un adhésif 3 non irritant pour la peau comme les adhésifs polyacrylates décrits dans le brevet US 2884126, lui-même recouvert d'un support protecteur 4 antiadhérent constitué par exemple d'un papier siliconé.

Le pansement 1 est replié sur lui-même en deux parties égales 1a et 1b du côté du support protecteur 4 selon une ligne de pliage 5 transversale.

La partie 4a du support protecteur 4 correspondant à la partie 1a du pansement est pourvue sur toute sa largeur d'une ligne de découpe 6 parallèle à la ligne de pliage 5 et située à proximité de cette dernière.

La zone 7 du support protecteur 4a qui est comprise entre les lignes de découpe 6 et de pliage 5 est collée à la zone correspondante de la partie 4b du support 4 au moyen d'un adhésif 8 hot-melt ou thermodurcissable de telle manière que la zone 7 adhère plus fortement à la zone correspondante du support 4b qu'au film plastique 2.

Une languette 10 est collée au bord de la découpe 6 de manière à faciliter le décollement de la partie 4a complémentaire de la zone 7.

Lors du positionnement, selon les figures 2 et 3, les parties 1a et 1b du pansement sont dépliées.

Ainsi la partie 1b comporte le support protecteur 4b et la zone 7 qui est restée collée à celui-ci. La partie du film ainsi découverte, du fait que la zone 7 du support est restée collée à la partie 4b correspondante du support, est ensuite appliquée sur la peau en étant maintenue tendue par une traction exercée sur les extrémités des parties 1a et 1b. Puis le support 4a est retiré au moyen de la languette 10 et la partie 1a est appliquée en sa totalité sur la peau. Le support 4b ainsi que la zone 7 du support 4a sont ensuite retirés et la partie 1b du film est appliquée sur la peau.

## Revendications

1. Pansement adhésif (1) constitué d'un film plastique (2) recouvert, sur une face, d'un adhésif permanent (3) et d'un support (4) antiadhérent protégeant l'adhésif, caractérisé en ce que :
— le pansement (1) est replié sur lui-même en deux parties (1a, 1b), du côté du support protecteur (4), selon une ligne de pliage transversale (5),
— la partie (4a) du support protecteur correspondant à une des deux parties (1a) du pansement est munie d'une ligne de rupture (6) sensiblement parallèle à la ligne de pliage (5),
— la zone (7) du support (4) comprise entre la ligne de rupture (6) et la ligne de pliage (5) est pourvue sur sa face externe d'un adhésif (8) qui agit de manière que ladite zone (7) adhère plus fortement à la zone correspondante de l'autre partie (4b) du support qu'au film plastique (2).

2. Pansement selon la revendication 1, caractérisé en ce que la ligne de rupture (6) est une ligne de découpe.

3. Pansement selon la revendication 2, caractérisé en ce que l'adhésif (8) est de type thermodurcissable ou hot-melt.

4. Pansement selon la revendication 1, caractérisé en ce qu'une languette (10) est fixée au bord de la ligne de rupture (6) de manière à faciliter le décollement de la partie (4a) du support, complémentaire de la zone (7).

## Claims

1. Adhesive dressing (1) consisting of a plastic film (2) covered on one face with a permanent adhesive (3) and with an anti-adhesive support (4) protecting the adhesive, characterised in that :
— the dressing (1) is folded back on itself in two portions (1a, 1b), on the side of the protective support (4), along a transverse fold line (5),
— the portion (4a) of the protective support corresponding to one of the two portions (1a) of the dressing is provided with a breaking line (6) approximately parallel to the fold line (5),
— the area (7) of the support (4) between the breaking line (6) and the fold line (5) is provided on its outer face with an adhesive (8) which works in such a way that said area (7) adheres more strongly to the corresponding area of the other portion (4b) of the support than to the plastic film (2).

2. Dressing according to claim 1, characterised in that the breaking line (6) is a stamped line.

3. Dressing according to claim 2, characterised in that the adhesive (8) is of the thermosetting or hot-melt type.

4. Dressing according to claim 1, characterised in that a tab (10) is fixed to the edge of the breaking line (6) so as to facilitate release of the support portion (4a) complementary to area (7).

**Patentansprüche**

1. Klebeverband (1), bestehend aus einem Kunststoffilm (2), der auf seiner einen Seite mit Dauerklebstoff (3) beschichtet ist und einem nicht haftenden Film (4) zum Schutz des Klebstoffes, dadurch gekennzeichnet, daß
— der Verband (1) entlang einer quer verlaufenden Faltlinie (5) dergestalt zusammengeklappt ist, daß die beiden Teile (1a, 1b) des Verbandes (1) mit den Seiten, auf denen sich der Schutzfilm (4) befindet, aufeinander zu liegen kommen,
— ein Teil (4a) des Schutzfilms (4), der zu einem (1a) der beiden Teile des Verbandes (1) gehört, mit einer Bruchlinie (6) versehen ist, die deutlich parallel zu der Faltlinie (5) verläuft,
— der Schutzfilm (4) in einem Bereich (7) zwischen der Bruchlinie (6) und der Faltlinie (5) auf seiner Außenseite mit Klebstoff (8) versehen ist, der so beschaffen ist, daß der erwähnte Bereich (7) stärker an dem entsprechenden anderen Teil (4b) des Schutzfilms (4) haftet als an dem Kunststoffilm (2).

2. Verband nach Anspruch 1, dadurch gekennzeichnet, daß die Bruchlinie (6) eine Einschnittslinie ist.

3. Verband nach Anspruch 2, dadurch gekennzeichnet, daß der Klebstoff (8) wärmehärtend ist oder heißklebend (Hot-Melt).

4. Verband nach Anspruch 1, dadurch gekennzeichnet, daß eine Lasche (10) dergestalt am Rande der Bruchlinie (6) befestigt ist, daß durch besagte Lasche ein Abziehen des Schutzfilmteils (4a) erleichtert wird, der komplementär zu dem Bereich (7) ist.

**FIG.1**

**FIG.2**

**FIG.3**